# EUROPÄISCHE PATENTSCHRIFT

(11) **EP 0 613 553 B1**
(45) Veröffentlichungstag und Bekanntmachung des Hinweises auf die Patenterteilung: **31.07.1996**
(21) Anmeldenummer: 92923523.2
(22) Anmeldetag: 17.11.1992
(51) Int. Cl.: G01N 27/12, G01N 33/00

(54) **VERFAHREN ZUR BESTIMMUNG VON KLEINEN MENGEN AN KOHLENMONOXID UND STICKOXIDEN IN GASGEMISCHEN**
METHOD FOR DETERMINING SMALL QUANTITIES OF CARBON MONOXIDE AND NITROGEN OXIDES IN GASEOUS MIXTURES
PROCEDE DE DETERMINATION DE PETITES QUANTITES D'OXYDE DE CARBONE ET D'OXYDES NITRIQUES DANS LES MELANGES GAZEUX

(30) Priorität: 22.11.1991 DE 4138369
(43) Veröffentlichungstag der Anmeldung: 07.09.1994
(73) Patentinhaber: ROBERT BOSCH GMBH, 70442 Stuttgart (DE)
(72) Erfinder: HUTH, Gerhard, D-7016 Gerlingen (DE); KLING, Anton, D-7016 Gerlingen (DE); BARESEL, Detlef, D-7000 Stuttgart 70 (DE)
(86) Internationale Anmeldenummer: DE9200958
(87) Internationale Veröffentlichungsnummer: WO9310441

(56) Entgegenhaltungen:
- EP-A- 0 077 724
- WO-A-92/13270
- DE-A- 2 603 785
- DE-A- 2 713 622

## Beschreibung

Die Erfindung betrifft ein Verfahren zur Bestimmung von kleinen Mengen an Kohlenmonoxid und an Stickoxiden in Sauerstoff enthaltenden Gasgemischen mit Hilfe eines Sensors, dessen elektrischer Widerstand sich mit der Konzentration an Kohlenmonoxid und Stickoxiden in bestimmter Weise verändert.

### Stand der Technik

In Abgasen aus Verbrennungsprozessen sind häufig neben Stickstoff, Sauerstoff und unverbrannten Kohlenwasserstoffen sowie durch Verbrennung entstandenem Kohlendioxid kleine Mengen an Kohlenmonoxid und an Stickoxiden enthalten. Diese Gase sind Schadstoffe im Sinne des Umweltschutzes und müssen daher so weit wie möglich entfernt werden. Dazu ist es wichtig, über Verfahren zu verfügen, die den Gehalt der Gasgemische an diesen Gasen in einfacher Weise und hinreichend genau zu bestimmen gestatten. Derartige Verfahren sind z.B. aus EP-A-77724 und DE-A-27 13 622 bekannt.

### Vorteile der Erfindung

Das Verfahren nach der Erfindung gestattet, jedes der beiden genannten Gase in einfacher Weise mit einer für alle praktischen Zwecke hinreichender Genauigkeit zu bestimmen. Da die sensoren schnell ansprechen, sind die Werte sofort verfügbar. Das Verfahren läßt sich unschwer automatisieren, wodurch es wohlfeil und weniger abhängig von Bedienungsfehlern wird. Die Sensoren haben eine lange Lebenszeit, auch bei ständig wechselnder Zusammensetzung der Gasgemische und bei häufigem Wechsel der Meßtemperatur, so daß das Verfahren sich hervorragend für den Dauerbetrieb eignet. Die Sensoren sind klein in ihren Abmessungen, haben also nur geringen Platzbedarf.

### Zeichnung

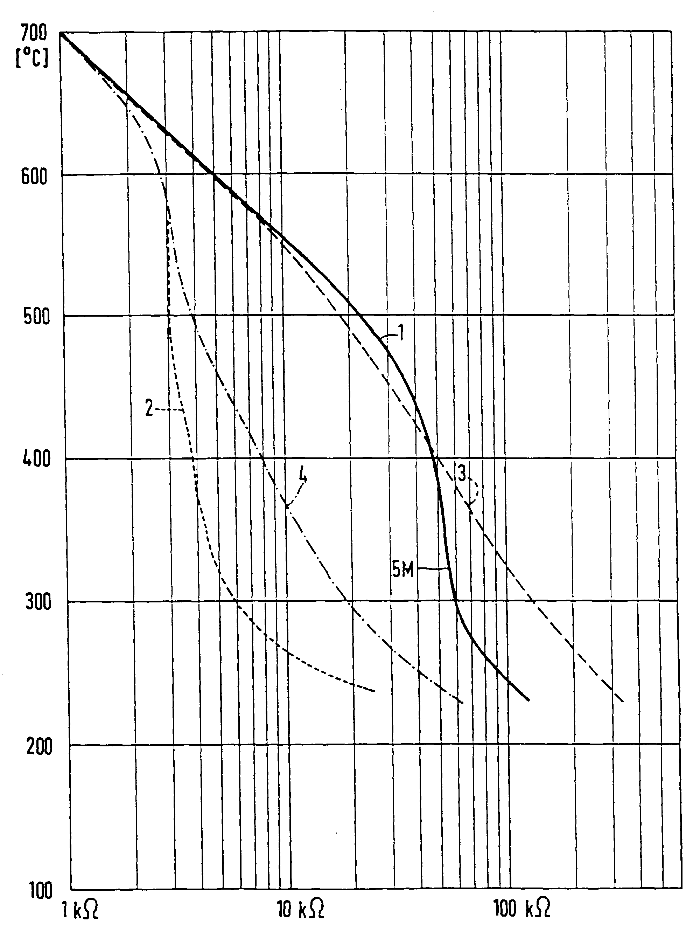

Die Zeichnung gibt in halblogarithmischer Auftragung die Widerstands-Temperatur-Funktion eines nach dem Verfahren der Erfindung arbeitenden Sensors für verschiedene Gasgemische wieder, nämlich
1 20 % Sauerstoff, 80 % Stickstoff
2 20 % Sauerstoff, 200 ppm Kohlenmonoxid, Rest Stickstoff
3 20 % Sauerstoff, 50 ppm Sickstoffmonoxid, Rest Stickstoff
4 20 % Sauerstoff, 200 ppm Kohlenmonoxid, 50 ppm Stickstoffmonoxid, Rest Stickstoff
5 wie 1.

Dabei sind hier und im folgenden, soweit nicht anders angegeben, die Prozentangaben Volumenprozente und die Teile ( = parts ) in den ppm-Angaben Volumenteile.

### Beschreibung der Erfindung

Die aufgeführten Vorteile werden durch das Verfahren nach der Erfindung erzielt, wie es in den Patentansprüchen umschrieben ist.

Das Verfahren eignet sich besonders für die Bestimmung von Kohlenmonoxid und von Stickoxiden in mageren, d.h. wenig Kohlenmonoxid und Stickoxide sowie vergleichsweise viel Sauerstoff enthaltenden Gasgemischen. Der Gehalt an Kohlenmonoxid liegt im allgemeinen zwischen 1 und 10 000 ppm, insbesondere zwischen 10 und 1 000 ppm, der an Stickoxiden ( d.h. Stickstoffmonoxid und Stickstoffdioxid ) zwischen 0,1 und 5000 ppm, insbesondere zwischen 1 und 500 ppm. Der Sauerstoffgehalt liegt im allgemeinen zwischen 0,1 und 15 %, insbesondere zwischen 1 und 6 %. Der Rest der Gasgemische besteht überwiegend aus Stickstoff sowie Kohlendioxid. Kleinere Mengen an unverbrannten Kohlenwasserstoffen und an Schwefeldioxid können zugegen sein, ohne die Bestimmung von Kohlenmonoxid und Stickoxiden zu stören.

Für das Verfahren eignen sich besonders Sensoren, die für die beiden zu bestimmenden Gase eine Widerstands-Temperatur-Charakteristik nach der Zeichnung aufweisen. Das trifft in hohem Maße für die Sensoren nach der deutschen Patentanmeldung P 41 00 915.0 zu. Dabei handelt es sich um Sensoren auf der Basis eines n-leitenden Metalloxids, wie Zinn(IV)oxid, das derart mit anderen Metalloxiden dotiert ist, daß die Oxidation des Kohlenmonoxids zu Kohlendioxid mit verringerter Geschwindigkeit abläuft. Geeignet ist z.B. Zinn(IV)oxid, das mit 0,01 bis 0,2 Mol% Magnesiumoxid, 0,01 bis 0,2 Mol% Palladiumoxid und 0.001 bis 0,1 Mol% Tantal(V)-oxid dotiert ist.

Das Verfahren nach der Erfindung gründet sich auf die Beobachtung, daß bei Verwendung geeigneter Sensoren die Widerstands-Temperatur-Kurven jedes einzelnen der beiden genannten Gase ( d.h. in Abwesenheit des jeweils anderen Gases ) sowie die Kurve der Kombination beider Gase im zu analysierenden Gasgemisch zueinander sowie in bezug auf die Referenzkurve ( Gasgemisch ohne die beiden zu bestimmenden Gase) eine bestimmte, die Messungen begünstigende Lage aufweisen. Das ist der Fall, wenn die Kurve eines der beiden zu bestimmenden Gase über weite Temperaturbereiche von der Referenzkurve deutlichen Abstand hält und die Kurve der Kombination der beiden Gase im Gasgemisch in einem Teil dieses Temperaturbereichs der Kurve dieses einen der zu bestimmenden Gases nahekommt ( oder gar praktisch mit ihr zusammenfällt ) und in einem anderen Teil des Temperaturbereiches deutlichen Abstand von ihr hält.

Diese Bedingungen werden von den erwähnten Sensoren nach der deutschen Patentanmeldung P 41 00 915.0 in günstiger Weise erfüllt. Die Messungen, die die Kurven nach der Zeichnung ergaben, wurden mit einem Sensor ausgeführt, dessen aktive Komponente die folgende Zusammensetzung hatte :
Zinn(IV)-oxid mit
0,1 Mol% Magnesiumoxid
0,1 Mol% Palladiumoxid
0,005 Mol% Tantal(V)-oxid

Für diesen Sensor gelten die Werte der Abbildung, die mit einem Meßstrom von 1 mA gemessen wurden. Kurve 1 ist die Referenzkurve für "synthetische" Luft, sie wurde mit aufsteigender Temperatur gemessen. Kurve 5, in demselben Medium, aber mit absteigender Temperatur gemessen, fällt mit Kurve 1 zusammen. Es wird also keine die Messungen verfälschende Hysterese beobachtet. Kurve 2 zeigt durch deutliche Abweichungen von der Kurve 1, daß kleine Mengen Kohlenmonoxid im Bereich zwischen etwa 250°C und etwa 600°C erhebliche Widerstandsänderungen hervorrufen. Dagegen ist der Einfluß des Stickstoffmonoxids allein zumindest im Temperaturbereich zwischen etwa 400°C und etwa 700°C gering, die Kurven 1 und 3 fallen hier praktisch zusammen. Kurve 4 gibt die Messungen mit kleinen Mengen Kohlenmonoxid und Stickstoffmonoxid wieder, sie kommt im Temperaturbereich zwischen etwa 500°C und etwa 600°C der Kohlenmonoxid-Kurve 2 nahe. In diesem Bereich ist die Widerstandsänderung durch Stickstoffmonoxid vergleichsweise gering, er eignet sich also für eine hinreichend genaue Bestimmung von Kohlenmonoxid durch nur eine Messung in dem genannten Temperaturbereich. Dagegen sind die Kurven 2 und 4 im Temperaturbereich von etwa 250 °C bis etwa 400°C deutlich verschieden, d.h. beide Gase tragen in erheblichem Umfang zur Widerstandsänderung bei. Man kann daher bei einer Temperatur in diesem Bereich einerseits die Widerstandsänderung durch das Kohlenmonoxid allein, dessen Konzentration aus der ersten Messung bei höherer Temperatur bekannt ist, bestimmen und andererseits die Widerstandsänderung durch beide Gase messen. Die Abweichung beider Meßwerte ist ein Maß für die Konzentration an Stickstoffmonoxid.

Man kann also Bestimmung beider Gase nebeneinander in einfacher Weise durchführen, nämlich durch 3 Messungen bei 2 verschiedenen Temperaturen. Da die Sensoren sehr schnell ansprechen ( im Bereich Millisekunden ), ist der Zeitbedarf gering. Wenn eine Vielzahl von Messungen über einen längeren Zeitraum zu erwarten ist, kann man mit Hilfe eines entsprechenden Rechenprogrammes die gemessenen Widerstandswerte ohne weiteren intellektuellen Aufwand in die gewünschten Konzentrationen umwandeln. Dazu bedarf es einer Reihe von Messungen der Widerstandsänderungen der Gasgemische mit dem einen und mit beiden der zu bestimmenden Gase bei der gewünschten Meßtemperatur, die einen eindeutigen Zusammenhang zwischen den Meßwerten und der Konzentration an Stickoxid herstellen.

Die Genauigkeit der Messungen läßt sich noch erhöhen, wenn man zusätzlich einen Inhibitor einsetzt, der die das Meßergebnis verfälschende Reaktion

CO + NO = CO₂ + 1/2 N₂

verlangsamt. Derartige Inhibitoren sind z.B. Mangan(II)oxid und Chrom(III)-oxid. Sie können dem Zinn(IV)-oxid des Sensors zugefügt oder als gesonderte Inhibitor-Schicht vorgelagert werden. Man benötigt dann zwei Sensoren, den einen mit, den anderen ohne den Inhibitor, deren Signale getrennt aufgefangen und verarbeitet werden müssen. Ein entsprechendes Rechenprogramm liefert dann die bereinigten Konzentrationen der beiden Gase, die den Fehler durch die erwähnte Reaktion berücksichtigen.

## Patentansprüche

1. Verfahren zur Bestimmung von kleinen Mengen an Kohlenmonoxid und Stickoxiden in Sauerstoff enthaltenden Gasgemischen mit Hilfe eines Sensors, dessen elektrischer Widerstand sich bei erhöhter Temperatur mit der Konzentration an Kohlenmonoxid und Stickoxiden verändert, dadurch gekennzeichnet, daß man (a) die durch den Kontakt des Sensors mit dem zu analysierenden Gasgemisch verursachte Widerstandsänderung bei einer ersten Temperatur T₁ in einem Temperaturbereich mißt, in dem die Widerstandsänderung durch das eine der beiden zu bestimmenden Gase erheblich und die durch das andere der beiden Gase verursachte vergleichsweise gering ist, und so die Konzentration dieses einen der beiden Gase bestimmt, (b) die Widerstandsänderung durch dieses Gas in der zuvor bestimmten Konzentration bei einer zweiten Temperatur T₂ in einem Temperaturbereich, in dem beide Gase in erheblichem Umfang zur Widerstandsänderung beitragen, mißt, (c) bei dieser zweiten Temperatur T₂ die Widerstandsänderung mißt, die durch das Gasgemisch mit beiden zu bestimmenden Gasen darin verursacht wird, und (d) aus der Differenz der Widerstandsänderungen nach (b) und (c) die Konzentration des anderen der zu bestimmenden Gase ermittelt.

2. Verfahren nach Anspruch 1, dadurch gekennzeichnet, daß man einen Sensor auf Basis eines n-leitenden Metalloxids verwendet, das derart mit anderen Metalloxiden dotiert ist, daß die Oxidation des Kohlenmonoxids zu Kohlendioxid mit verringerter Geschwindigkeit abläuft.

3. Verfahren nach Anspruch 2, dadurch gekennzeichnet, daß man einen Sensor auf Basis von Zinn(IV)-oxid verwendet, das mit 0.01 bis 0,02 Mol% Magnesiumoxid, 0,01 bis 0,2 Mol% Palladiumoxid und 0,001 bis 0,1 Mol% Tantal(V)-oxid dotiert ist.

4. Verfahren nach den Ansprüchen 1 bis 3, dadurch gekennzeichnet, daß man zusätzlich einen Inhibitor einsetzt, der die Reaktion
CO + NO = CO₂ + 1/2 N₂
verlangsamt.

## Claims

1. Method for the determination of small amounts of carbon monoxide and nitrogen oxides in oxygen-containing gas mixtures with the aid of a sensor whose electrical resistance changes at elevated temperature with the concentration of carbon monoxide and nitrogen oxides, characterized in that (a) the resistance change caused by the sensor being in contact with the gas mixture to be analysed is measured at a first temperature T₁ in a temperature range within which the resistance change caused by one of the two gases to be determined is considerable and that caused by the other of the two gases is comparatively small, and in this way the concentration of the one of the two gases is determined, (b) the resistance change due to said gas at the previously determined concentration is measured at a second temperature T₂ in a temperature range within which both gases contribute to a considerable extent to the resistance change, (c) at said second temperature T₂ the resistance change is measured which is caused by the gas mixture containing both gases to be determined, and (d) based on the difference of the resistance changes according to (b) and (c) the concentration of the other gas to be determined is obtained.

2. Method according to Claim 1, characterized in that a sensor is used which is based on an n-type metal oxide which is doped with other metal oxides in such a way that the oxidation of the carbon monoxide to carbon dioxide proceeds at a reduced rate.

3. Method according to Claim 2, characterized in that a sensor is used which is based on tin(IV) oxide which is doped with from 0.01 to 0.02 mol% of magnesium oxide, from 0.01 to 0.2 mol% of palladium oxide and from 0.001 to 0.1 mol% of tantalum(V) oxide.

4. Method according to Claims 1 to 3, characterized in that in addition an inhibitor is employed by which the reaction
CO + NO = CO₂ + 1/2 N₂
is retarded.

## Revendications

1. Procédé pour la détermination de petites quantités d'oxyde de carbone et d'oxydes nitreux dans des mélanges gazeux contenant de l'oxygène à l'aide d'un détecteur, dont la résistance électrique se modifie à température élevée avec la concentration en oxyde de carbone et en oxydes nitreux, caractérisé en ce que :
a) on mesure la modification de résistance causée par le contact du détecteur avec le mélange gazeux à analyser à une première température T₁ dans une zone de température, dans laquelle la modification de la résistance causée par l'un des deux gaz à déterminer est importante et celle causée par l'autre des deux gaz est comparativement faible, et ainsi on détermine la concentration de ce premier des deux gaz,
b) on mesure la modification de résistance causée par ce gaz dans la concentration déterminée précédemment à une deuxième température T₂, dans une zone de température, dans laquelle les deux gaz contribuent dans une mesure importante à la variation de résistance,
c) on mesure à cette deuxième température T₂ la variation de résistance, qui est causée par le mélange gazeux avec les deux gaz à déterminer, et
d) de la différence des modifications de résistance selon (b) et (c) on détermine la concentration de l'autre gaz à déterminer.

2. Procédé selon la revendication 1, caractérisé en ce qu'on utilise un détecteur à base d'un oxyde métallique n-conducteur, qui est doté d'autres oxydes métalliques, de manière que l'oxydation de l'oxyde de carbone se termine avec une vitesse réduite en gaz carbonique.

3. Procédé selon la revendication 2, caractérisé en ce qu'on utilise un capteur à base d'oxyde d'étain (IV), qui est doté de 0,01 à 0,02% molaire d'oxyde de magnésium, de 0,01 à 0,2% molaire d'oxyde de palladium et de 0,001 à 0,1% molaire d'oxyde de tantale (V).

4. Procédé selon les revendications 1 à 3, caractérisé en ce qu'on utilise en supplément un inhibiteur qui ralentit la réaction :
CO + NO = CO₂ + 1/2 N₂
